# EUROPEAN PATENT APPLICATION

(11) **EP 4 481 755 A1**
(43) Date of publication of application: **25.12.2024**
(21) Application number: 24183261.7
(22) Date of filing: 20.06.2024
(51) Int. Cl.: G16H 20/40, G16H 40/20

(54) **APPARATUS AND SYSTEM FOR IMPROVING TROUBLESHOOTING IN MEDICAL SYSTEMS**

(30) Priority: 21.06.2023 US 202363522249 P; 28.05.2024 US 202418675338
(71) Applicant: Biosense Webster (Israel) Ltd., 2066717 Yokneam (IL)
(72) Inventor: MALKA, Yakir, 2066717 Yokneam (IL); AM-SHALEM, Shlomo, 2066717 Yokneam (IL)
(74) Representative: Carpmaels & Ransford LLP

(57) **Abstract**

A method, apparatus and computer program product, the method comprising: obtaining an audio stream comprising speech by a user of a medical system during an operation involving the medical system, wherein the operation is performed upon a subject; obtaining at least one data stream from equipment associated with the medical system; detecting within the audio stream an indicator to a problem, the indicator occurring at least during a first time window; selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and outputting the audio segment and the at least one data segment.

## Description

### CROSS-REFERENCE TO RELATED APPLICATIONS

The present application claims priority from U.S. Provisional Patent Application Ser. No. 63/522,249, filed June 21, 2023, entitled "APPARATUS AND SYSTEM FOR IMPROVING TROUBLESHOOTING IN MEDICAL SYSTEMS" which is incorporated herein by reference.

### FIELD OF THE DISCLOSURE

The present disclosure relates to medical systems in general, and to a system and method for improving troubleshooting in medical systems, in particular.

### BACKGROUND OF THE DISCLOSURE

Many advanced systems, and in particular medical systems, in addition to their main functionality also record continuously and in real-time the user's and the system's activities.

For example, the user's actions may be logged, including actions associated with user interface devices such as a keyboard or a pointing device, actions associated with the system and the relevant parameters thereof, such as applying energy to a tissue during an electrophysiological (EP) mapping and ablation operation and others. In addition, certain events bookmarked by a user may also be logged.

Additionally, raw data obtained by the system may be collected and logged, such as various digital samples of measurements taken by electrodes or other measuring devices, location recordings of devices used during the operation such as one or more catheters, or the like.

A further type of recorded data may include data processing results such as visualization of device paths or electrocardiogram (ECG) morphologic particularities, which may or may not include intermediate computation results.

Additionally, logs may be obtained from underlying software, including for example recordings of code execution traces programmed by the software developers, or the like for purposes such as debugging or performance monitoring, recording of system events, network message, or the like.

Due to the abovementioned data types and additional ones amounting to significant volumes recorded per time unit, and in particular per operation, searching for a specific event within this abundance of data, for example for troubleshooting purposes, is a challenging task.

Traditionally, the data is reviewed and such events are searched for when something goes wrong with the operation, and in particular when it is required for evaluating the physician's actions and compliance with the required medical standards.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of an exemplary medical environment, comprising a catheter-based electrophysiological (EP) mapping and ablation system, in accordance with some exemplary embodiments of the disclosure;
Fig. 2 is a pictorial illustration of various data sources and data extracted and recorded therefrom, in accordance with some exemplary embodiments of the disclosure;
Fig. 3 is a flowchart of steps in a method for troubleshooting a medical system, in accordance with some exemplary embodiments of the disclosure; and
Fig. 4 is a schematic block diagram of a computing platform for troubleshooting a medical system, in accordance with some exemplary embodiments of the disclosure.

### DETAILED DESCRIPTION OF EXAMPLES

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### OVERVIEW

In the disclosure below, the term "word" is to be broadly construed to cover any word or phrase comprising one or more words, syllables, or the like. The word may be in any language or dialect, and may include single syllables or combinations, or vocal gestures such as "uh-uh", "oh no", etc.

In the disclosure below, the term "stream", whether as associated with an audio stream or another data stream, is to be broadly construed to cover any sequence of digital data, received from a source at any frequency or time resolution and in any format. The data may be processed or otherwise used online as it is being received, or stored in a storage device and processed at a later time, for example as a file comprising all or part of the received data.

In many medical operations, a user such as a physician makes use of complex equipment. Two such examples include electrophysiology mapping and ablation system, and phacoemulsification system.

In the disclosure below, the term "problem" is to be broadly construed to cover any technical, software or other issues with the systems and equipment used by the physician or other staff members, and which may be investigated by the developers. The term "problem" may be used interchangeably with the term "troubleshooting event".

Huge amounts of data, including measurements, user actions, system actions, events of the underlying computing system, intermediate and final processing results and others may be collected during the operation. Typically, the information is used for monitoring or evaluating the user's actions, e.g., making sure that a physician took the right decisions and performed the correct actions at the right time.

As medical systems are generally complex, they are naturally prone to problems, including bugs, misusage problems, or others, and in particular during development or deployment of new systems or new versions thereof. Thus, it may be required to troubleshoot such problems.

However, as detailed above, the huge amount of collected data makes troubleshooting hard, as it is generally unknown whether and when a problem occurred, or when it was noticed by a user.

In accordance with some embodiments of the disclosure, in addition to recording all the data streams from the systems as is currently done, a microphone, an audio recorder, and user actions recorder may be used for capturing and recording audio and other data entered by the users, including the physician(s) and possibly additional stuff members, such as the clinical assistants (CAS). The audio may be recorded throughout the operation, or one or more parts thereof.

The recorded audio may be analyzed for detecting troubleshooting indicators by one of the recorded personnel members.

The indicators may be words indicating the existence of any problem, such as "uh-uh", "problem", "disabled", "not working", "the screen has frozen", "I cannot move the mouse", "the system is very slow", "the system is not responsive", "I cannot click that button"; "I cannot find that that option/button", "the button is disabled", "the button/option has disappeared", "I click but nothing happens", "the window/dialog has closed/disappeared", "I cannot move/minimize/maximize the window/pane", "The error/warning means nothing to me", "the performance is bad", "How can I erase/remove/solve that error/warning", "the system does not work and no error is displayed", "restart the system", or the like.

One or more words may also be domain-specific and may indicate a problem with the specific system. For example the following words or phrases may be relevant to electrophysiology mapping and ablation systems or its operation: "the ablation time/temperature/power seems wrong", "the force indication is wrong", "the catheter is stuttering", no connection with the piu/ultrasound machine/recording system", "maybe we should switch the piu off" "the annotation is incorrect", "the ECG monitor has frozen/stopped", "a point is missing", "a contour is missing", "the catheter visualization does not work", "I cannot save/archive/restore the study", or the like.

Further indications may include identification of problematic emotional states within the audio, such as stress, anger, worry, or the like.

In some embodiments, inappropriate words may also be searched for, since such words may indicate frustration with the system.

In some embodiments, the words may be recognized by fully transcribing the captured audio, and searching the transcription for the collection of problem-indicating words, whether general or specific.

In further embodiments, the words may be recognized using small vocabulary speech recognition techniques, in which only specific words are searched for.

The words may be searched for in a fuzzy manner, such that similar words or phrases may also be detected, for example using "we" rather than "I", different order of words in a phrase, or the like. For example, each detected word may be associated with a certainty degree, and only words identified with a certainty degree exceeding a threshold may be output for further handling.

Once an indicator has been detected, a time slot, comprising a first time window during which the word or emotional state was detected, a second time window preceding the first time window, and optionally a third time window following the first time window may be marked for investigation. In some embodiments, each of the first, second and third time windows may be of a predetermined (and possibly different) length, for example one second, ten seconds, one minute, ten minutes, fifteen minutes or the like. In some embodiments, the second time window may go back from the first time window until a predetermined event, such as the insertion of a catheter into the vein, arrival of the catheter to the heart, an ablation application, the beginning of the operation, or the like. In some embodiments, the third time window may extend from the first time window until a predetermined event, such as the catheter being pulled out of the heart, the end of the operation, or the like. The timing of the predetermined events may be obtained from a log of the operation, from another recording, from one or more words detected in the audio recording, or the like. In some embodiments, the preceding event may be related to an underlying executed program, such as spawning of a thread, activating a module, or the like. The third time slot may continue until a

Once the time slot including the first, second and third time windows is determined, it may also be applied to the other captured streams, including for example ongoing measurements, user actions, system actions, system events, network events, or the like, such that only the segments corresponding to the time slots thereof are further handled.

In some embodiments, any identifying details of the subject of the operation may be concealed from the audio stream and from the data streams, within the time slot. For example, the patient's or the physician's name may be concealed if mentioned within the audio segment, removed from any data segment or from screen capture, or the like, to protect their anonymity and privacy. In some embodiments, informative details, such as sex or age of the subject (for example reference to the patient as "he" or "she") may be maintained or concealed, according to regulations, sensitivity, or the like.

The parts of the audio and data streams corresponding to the time slot may then, for example immediately or at any time later, be provided to another system or to a person in charge for troubleshooting the issue, for example determining whether there was indeed a problem, what led to the problem, how the situation is to be prevented, or the like. In some embodiments, the system may be fixed and updated for preventing such situations. In other embodiments, the user may receive guidance on how the situation is to be prevented, or the like.

The disclosure thus provides for automatic detection of troubleshooting situations during an operation, wherein the situations may involve a problem with the used equipment, and providing relevant audio and data information to a person or a system in charge of troubleshooting. The disclosure thus enables troubleshooting of problematic situations, such that the situation may be eliminated or corrected, either by fixing the system or by educating the users.

The disclosure also provides for effective and efficient troubleshooting since it is not required to review the whole body of data collected during the operation. A person or system in charge of troubleshooting only needs to review the relevant time periods when the user noticed the problem, and preceding and/or consequent time windows.

### SYSTEM DESCRIPTION

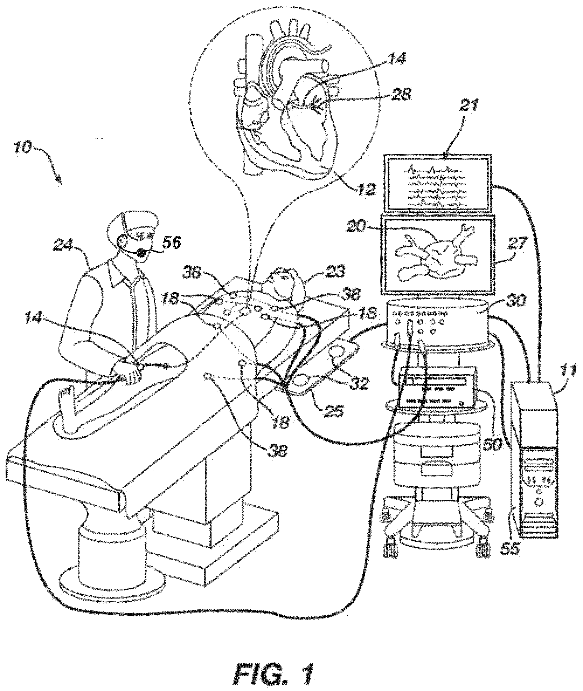

Reference is made to Fig. 1 showing an example catheter-based electrophysiology mapping and ablation system 10. It is however appreciated that the disclosure is not limited to such system, and may also be used with any other type of medical system, such as phacoemulsification systems or others.

Exemplary system 10 may include one or more catheters, which may be percutaneously inserted by a physician 24 through the vascular system of a patient 23 into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters, including for example catheter 14, may include catheters dedicated for sensing Intracardiac Electrogram (IEGM) signals, catheters dedicated for ablating and/or catheters for both sensing and ablating. Physician 24 may place a distal tip 28 of catheter 14 in contact with the heart wall for ablating a target site in heart 12.

Catheter 14 may be an exemplary catheter to be used with an irreversible electroporation (IRE)/pulsed field ablation (PFA) system. Distal tip section 28 of catheter 14 may comprise one or more electrodes connected by wires running through catheter 14 to console 30.

Once the distal end of catheter 14 has reached the target location within heart 12, physician 24 may further manipulate catheter 14 to place one or more electrodes disposed over distal tip 28 in contact with the target location, such as the ostium of the pulmonary vein.

The proximal end of catheter 14 receives energy from IRE ablation energy generator 50 through control console 30. IRE power source 50 may produce but is not limited to PFA energy, including monopolar, bipolar or a combination thereof of high-voltage DC pulses, as may be used to effect irreversible electroporation (IRE). Bipolar pulses may be applied between one or more pairs of electrodes.

Distal tip 28 section may comprise one or more sensors, such as force sensors, for sensing the force applied by the electrodes on the heart wall. The force sensor may sense and report the total force applied by the electrodes of distal tip 28 on the heart wall, which may be calculated upon the forces applied by each electrode touching the hear wall on the heart wall.

Distal tip 28 section may comprise position sensors for tracking the position and orientation of the distal, or other sensors. The position sensor may be a magnetic-based position sensor including three magnetic coils for sensing three-dimensional (3D) position and orientation. The magnetic based position sensor may be operated together with a location pad 25 including a plurality of magnetic coils 32 configured to generate magnetic fields in a predefined working volume. Real time position of distal tip 28 or any of the electrodes of catheter 14 may be tracked based on magnetic fields generated with location pad 25 and sensed by the magnetic based position sensor. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The readings from the force sensors, position sensors, or other sensors may be reported continuously, for example as a digital stream.

System 10 may include one or more electrode patches 38 positioned for skin contact on patient 23 to establish location reference for location pad 25 as well as impedance-based tracking of the electrodes. For impedance-based tracking, electrical current is directed to the electrodes and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 may record and display electrograms 21 captured with body surface ECG electrodes 18 and intracardiac electrograms (IEGM) captured with a corresponding catheter. Recorder 11 may include pacing capability for pacing the heart rhythm and/or may be electrically connected to a standalone pacer.

Patient interface unit (PIU) 30 is an interface configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 may include memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, (4) displaying on display device 27 sites of interest such as places where ablation energy has been applied, and (5) displaying on display device 27 a current PFA index 51, calculated upon the force and location of catheter 14. It is appreciated that the functions mentioned above are exemplary only, and a different set of functions may be provided to a user. One commercial product embodying elements of system 10 is available as the CARTO^{™} 3 System, available from Biosense Webster, Inc., 31A Technology Drive, Irvine, CA 92618.

System 10 may include one or more microphone 56 for capturing speech by physician 24. Microphone 56 may be head mounted on the head of physician 24, embedded within workstation 55, fixed within the room, or located anywhere else within the system or its environment. Microphone 56 may be wired or wirelessly connected to a device such as workstation 55 or another device adapted to record audio captured by microphone 56.

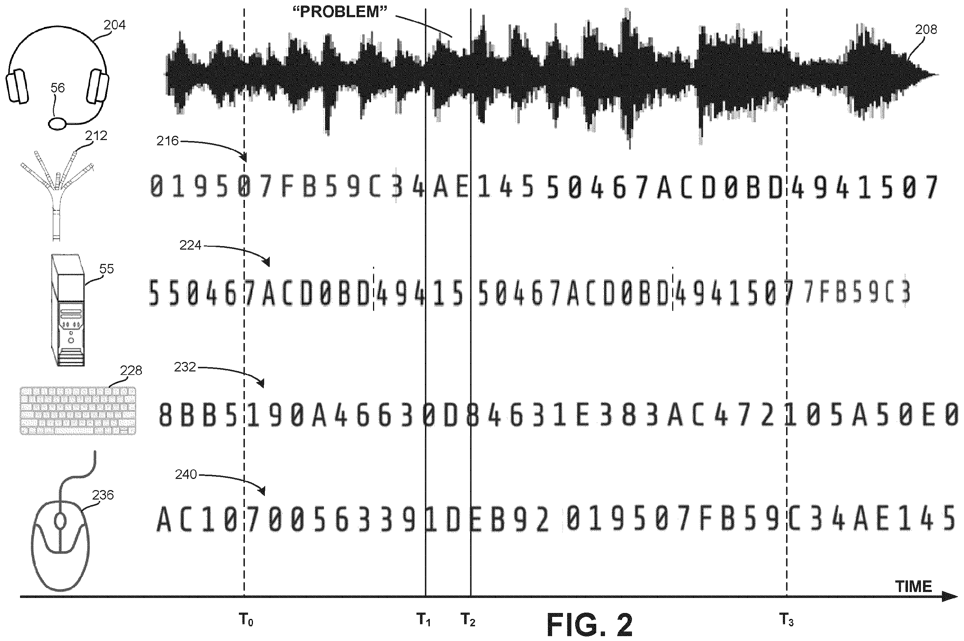

Referring now to Fig. 2, showing a partial exemplary pictorial illustration of the data collected during an operation, in accordance with some exemplary embodiments of the disclosure.

Fig. 2 shows microphone 56, which may or may not be connected to a headset 204, embedded within workstation 55, or the like. Microphone 56 may output a digital signal 208.

One or more electrodes 212 of a catheter may each output one or more digital streams 216, indicating for example ongoing pressure exerted by the electrode on the tissue wall, ongoing position measurement, or the like.

The hardware or software of workstation 55 or another computer platform associated therewith may also output a digital stream 224, comprising system events, interrupts, or the like.

Each of keyboard 228 and pointing device 236, such as a mouse, a touchscreen, a joystick, or the like, associated with workstation 55 may also output a digital stream, 232 and/or 240, respectively.

It is appreciated that the displayed audio streams are exemplary only, and that fewer, additional, or different data streams may be provided and used.

A problem may then be searched for within the audio stream, or within any of the data streams. For example, an audio analysis module, which may be embedded within workstation 55 or in another remote or collocated computing device, may receive and analyze audio stream 208 to search for words indicating a problem.

The audio analysis module may apply full transcription to audio stream 208, and search the transcription for a list of predetermined words. Additionally or alternatively, the audio analysis module may perform small vocabulary speech recognition and search specifically for the words from the predetermined list.

Additionally or alternatively, audio analysis module may perform emotional state analysis of audio stream 208 in order to recognize points in time in which the speaker's emotional state seems stressful, worried, angry, or the like.

In the example of Fig. 2, the word "problem" has been detected by the audio analysis module between time T₁ and time T₂.

In response to detecting the word (or the emotional state), at least a point in time which is earlier than T₁, for example T₀, may be determined.

In some embodiments T₀ may be selected arbitrarily, for example a predetermined period of time prior to T₁.

In further embodiments, T₀ may be selected upon a recognizable event preceding T₁, such as the insertion of a catheter, a computerized event reported by workstation 55 such as network problem, as user interface event, or the like.

The preceding events may be searched for within the audio stream, within any of the data streams, such as data stream 224, 232, or 240 or received from an external source.

Similarly, a point in time T₃ which is subsequent to T₂ may be selected. T₃ may be selected arbitrarily, for example 1 minute, 10 minutes, 15 minutes or the like after T₂. Alternatively, T₃ be identified when an event occurs as detailed above, at the end of the operation, or the like.

Optionally, identifying details of the patient, the physician, or another entity may be concealed within the audio and within the data streams between T₀ and T₃. For example the patient's name being said may be concealed within the audio, the patient's printed name may be concealed within any of the data streams, or the like. It is appreciated that the patient and physician names or any other identifying detail of any of the participants in the operation are input at the beginning of the operation and may also be available in the computerized systems of the organization or the operation. The details may thus be retrieved, searched for, and concealed within the audio, the recorded text as input through the keyboard, recorded screen, or in any other recorded information. The concealment may help ensure the privacy and anonymity of the physician, patient and any other involved person.

The segments of the audio and the data streams from T₀ to T₃ may then be provided to a user, a system, or the like.

It is appreciated that the devices and streams shown in Fig. 2 are exemplary only, and an actual system may output a different plurality of streams. Some exemplary data streams may include one or more of the following:
- Raw Electrocardiogram (ECG) digital sample recordings collected from ECG electrodes 18 attached to the patient body surface (for example at the limbs, chest or back) and from the intra-cardiac electrode catheters;
- ECG processed data, such as automatically detected ECG morphologic particularities, called "annotations", related to particular times in a normal or abnormal heart rhythm ECG;
- Intra-cardiac catheter navigation recordings, for example of the three-dimensional locations visited by catheters within the heart;
- Intra-cardiac catheter shaft visualization (for example as three dimensional geometrical curves);
- Catheter localization and visualization algorithmic computation results, whether intermediary or final, for algorithmic optimization, debugging and research;
- Localization based on electromagnetic signals, for example resulting from the artificial electromagnetic field generated by a low frequency signal generator placed under the patient table;
- Localization based on currents, for example synthesized micro generated artificially between the body surface patches and the catheter electrodes;
- Therapeutic radio frequency ablation or pulse field ablation applications, including time, duration, and repetition information recordings;
- Recording of the measurement of applied force, for example the mechanical force applied by the physician while pushing the catheter against the inner wall of the heart, to measure ECG or perform ablation;
- Recording of pacing events initiated by the physician. i.e., heart stimulation by application of electric pulses for inducing latent arrhythmia or for verifying ablation effectiveness to eradicate the arrhythmia;
- User action logger, including recording of actions performed by the user with the system user interface;
- User ultrasound frames or anatomical markers, for example recording of brief textual bookmarks typed into the system by the user;
- Software internal logger, including recording of code execution traces programmed by the software developers for debugging or for system computational performance monitoring purposes;
- Recording of the occurrence and disappearance of predefined system errors and warnings displayed to a user, such as a physician or a clinical assistant;
- Operating system event log, relating for example to the computation resource consumption;
- Operating system application or kernel crash event recording;
- Recording of ultrasound frames, and anatomical markers (contours) applied manually by the user or automatically by the software, in systems where a catheter is a equipped with an ultrasound transducer.

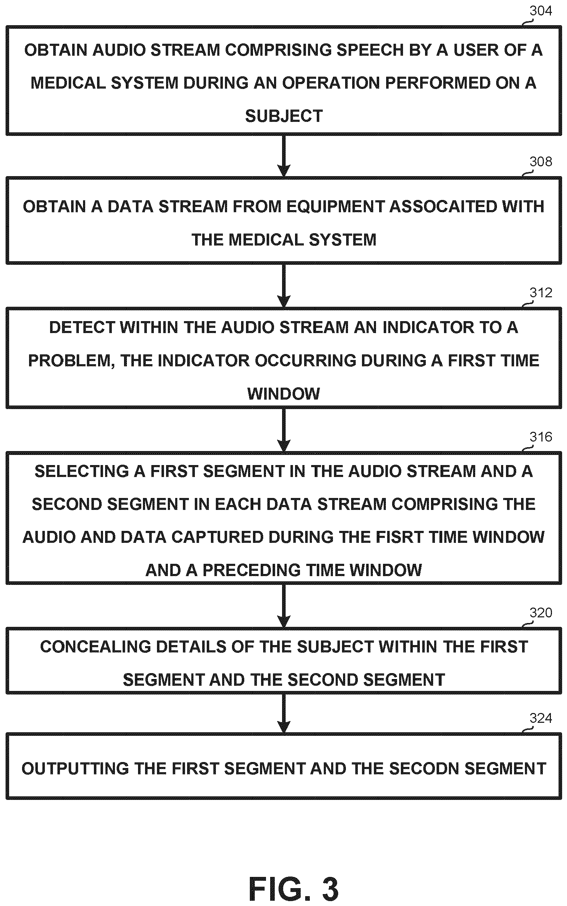

Referring now to Fig. 3, showing a flowchart of steps in a method for troubleshooting a medical system, in accordance with some exemplary embodiments of the disclosure.

On step 304, an audio stream comprising audio representing speech by one or more users such as a physician or clinical assistants during an operation performed over a subject may be received by a computing device. The audio stream may be received in real time, for example from an audio capture device such as a microphone, or after the stream has been stored on a storage device, for example in the form of a file. The audio stream may be captured by a microphone, whether in the form of a headset, ear pods, located anywhere in the room, or embedded in a workstation associated with the medical system.

On step 308, one or more data streams may be received from one or more sources associated with the operation. As detailed above, the data streams may be received from one or more sensors, from a computing platform comprising results of processing the received data, events associated with the computing platform or its peripherals and environment, or the like.

On step 312, the audio stream may be analyzed. In some exemplary embodiments, the analysis may include some preprocessing, for example noise reduction, silence or tone removal, speech separation into speech by different speakers, or the like. Following preprocessing, one or more troubleshooting indicators may be detected in the audio stream, for example occurring during a first time window. Some indicators may include the occurrence of words from a predetermined list, for example a list of words indicating a problem, and in particular a problem with the equipment of the medical system. The words may be detected by full transcribing the audio stream and searching for the list of words in the transcription, or applying small vocabulary speech recognition for the list of words. The words may be detected at a starting time, and last during a first time window. Other indicators may include a stressful, angry or similar emotional state identified by emotional state analysis of the audio stream, which may also indicate a problem. In some examples, the data streams may also be searched for such words, and the times at which the words are found may be indicated.

On step 316, an audio segment may be selected from the audio stream, and one or more data segments corresponding in time to the audio segment may be selected from one or more of the data streams. The audio segment and the data segments may comprise audio and data captured, respectively, on the first time window. The audio segment and the data segments may further comprise audio and data captured on a second time window preceding the first time window. The second time window may immediately precede the first time window. The second time window may start at the beginning of the operation or at a predetermined event, as detailed above.

The audio segment and the data segment may also comprise audio and data captured, respectively, on a third time window following the first time window. The third time window may immediately follow the first time window. The third time window may end at the end of the operation or at a predetermined event, as detailed above. In some embodiments, a user may indicate one or more of the boundaries of the first, second or third time windows, such that the user indicates where it is expected to find information for troubleshooting.

On step 320, the audio and data segments, where applicable, may be processed for concealing all identifying details of the subject of the operation, such as the subject's name or ID mentioned in the audio or coded in any of the data streams, or the like. In some embodiments, further details such as gender or age may also be concealed. The patient's spoken name may be concealed in the audio segment, and textual or binary identifying details such as printed name, gender, age, or the like may be concealed from the data segments.

On step 324, the audio segment and the at least one data segment may be output. The segments may be stored as one or more files, provided as streams over a communication channel, sent via a communication channel to a user such as a developer, or the like.

In some embodiments, customized software may assist a developer in analyzing the situation, by playing the segment of the audio stream, reproducing user actions such as keyboard, mouse, touch screen or other events, visualizing the data streams, for example showing the events or displaying textual data, displaying graphs, or the like.

Playback of the flow and events, including user actions, that took place before and after a problem has been indicated by the user, may increase the ability of a developer to reproduce bugs that may otherwise be considered irreproducible. The developer may then better analyze the situation and may be able to offer a solution.

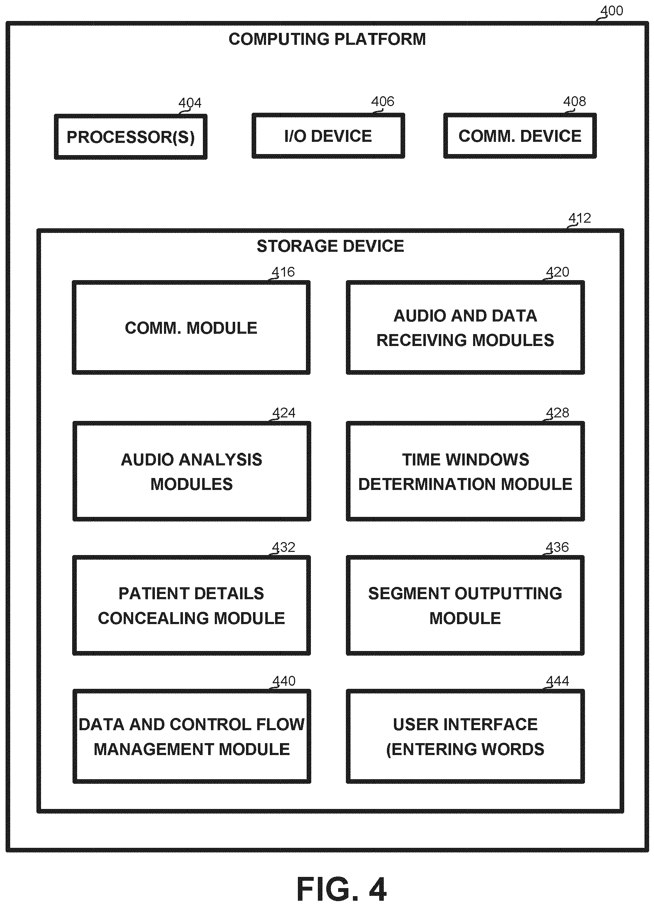

Referring now to Fig. 4, showing a block diagram of a computing platform 400 for troubleshooting a medical system, in accordance with some exemplary embodiments of the disclosure.

It will be appreciated that computing platform 400 may be embedded within workstation 55 or another computing platform of the system, but may also be a standalone computing platform or embedded elsewhere, on-premise or remote, and be in operative communication with workstation 55.

Computing platform 400 may be implemented as one or more computing platforms which may be operatively connected to each other. For example, one or more remote computing platforms, which may be implemented for example on a cloud computer. Other computing platforms may be a part of a computer network of the associated organization. In other embodiments, all the functionality may be provided by one or more computing platforms all being a part of the organization network.

Computing platform 400 may comprise one or more processors 404 located on the same computing platform or not, which may be one or more central processing units (CPU), graphics processing unit (GPU) microprocessors, electronic circuits, Integrated Circuits (IC) or the like. Processor 404 may be configured to provide the required functionality, for example by loading to memory and activating the software modules stored on storage device 412 detailed below.

Computing platform 400 may comprise Input/output (I/O) device 406, such as a microphone, a display, a keyboard, a touch screen, a mouse or another pointing device, a speaker, or the like. I/O Device 406 may be utilized to receive input from and provide output to a user such as a system developer, a system administrator, a physician, a clinical assistant or for example receive words to be detected provided as text or as audio, display data streams and play the audio stream, or the like.

Computing platform 400 may comprise a communication device 408 for communicating with other devices or other computing platforms, for example obtaining information from one or more sensors, obtaining operation information from a controller of the medical system, storing data on remote storage devices, or the like. Communication module 408 may be adapted to interface with any communication channel such as Local Area Network (LAN), Wide Area Network (WAN), cellular network or the like, and use any relevant communication protocol.

Computing platform 400 may comprise a storage device 412, such as a hard disk drive, a Flash disk, a Random Access Memory (RAM), a memory chip, or the like. In some exemplary embodiments, storage device 412 may retain program code operative to cause processor 404 to perform acts associated with any of the modules listed below, or steps of the method of Fig. 3 above. The program code may comprise one or more executable units, such as functions, libraries, standalone programs or the like, adapted to execute instructions as detailed below.

Alternatively or additionally, the provided instructions may be stored on non-transitory tangible computer-readable media, such as magnetic, optical, or electronic memory.

Storage device 412 may comprise communication module 416 for transmitting and receiving data to and from other systems or components, such as a control system, one or more sensors, external storage devices, a display system, or the like, through communication device 408. In particular, communication module 416 may be operative in receiving measurements from sensors associated with the electrodes and/or location information of the respective electrodes, processing results thereof, or the like.

Storage device 412 may comprise audio and data receiving module 420 for receiving audio stream comprising a voice of a user, and one or more data streams comprising data associated with the medical system or the computing system, as detailed in steps 304 and 308 of Fig. 3 above.

Storage device 412 may comprise one or more audio analysis modules 424 for detecting one or more words from a predetermined list of words, as detailed in association with step 312 of Fig. 3 above. Audio analysis modules 424 may also comprise emotional state analysis module for detecting stressful or other emotional state which can indicate a problem within the audio stream.

Storage device 412 may comprise one or more time window determination modules 428 for determining the time window preceding the segment in which the words, emotional state, or another indicator was detected. The time window may go back to the beginning of the operation, or to another predetermined event. Time window determination modules 428 may also be operative in determining the time window consequent to the segment in which the words, emotional state, or another indicator was detected. The time window may go forward until the end of the operation, or to another predetermined event.

Storage device 412 may comprise patient details concealing module 432 for concealing identifying details of the subject within the selected segments, as described in association with step 320 of Fig. 3 above.

Storage device 412 may comprise segment outputting module 436 for outputting the determined audio segments and data segments comprising audio and data of the first, second and third time windows, as described in association with step 324 of Fig. 3 above.

Storage device 412 may comprise data and control flow management module 440, for activating the modules above in the correct order and with the required input, for example determining the time windows after the indicators are found, which follows receiving the audio and data streams, or the like.

Storage device 412 may comprise user interface 444, for receiving input from and provide output to a user such as a system developer, a system administrator, a physician, a clinical assistant or the like. User interface 444 may, for example receive from a user words to be detected which may be provided as text or as audio, display data streams, play the audio stream, or the like.

Storage device 412 may further comprise or be in operative communication with storage space storing the audio and data streams as received, the parts of the audio and data streams containing the first, second and third time window, detected problems, or the like.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture instructions, machine instructions, machine dependent instructions, microcode, firmware instructions, state-setting data, or either source code or object code written in any combination of one or more programming languages, programming languages such as Java, C, C++, Python, or others. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### EXAMPLES

### Example 1

A method comprising:
obtaining during an operation involving a medical system, an audio stream comprising speech by a user using the medical system for performing the operation, wherein the operation is performed upon a subject;
obtaining at least one data stream from equipment associated with the medical system;
detecting within the audio stream an indicator to a problem, the indicator occurring at least during a time window;
selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and
outputting the audio segment and the at least one data segment.

### Example 2

The method according to example 1, wherein the indicator to the problem is detected by recognizing within the audio stream at least one word selected from a predetermined list of words.

### Example 3

The method according to example 1, wherein the indicator to the problem is detected by recognizing within the audio stream an emotional state indicative of a problem.

### Example 4

The method according to example 1, wherein the audio segment and the at least one data segment comprise, respectively, audio and data captured during a second time window preceding the time window.

### Example 5

The method according to example 4, wherein the second time window starts at a predetermined event.

### Example 6

The method according to example 4, wherein the second time window starts at a beginning time of the operation.

### Example 7

The method according to example 1, wherein the , the audio segment and the at least one data segment comprise, respectively, audio and data captured during a third time window consecutive to the time window.

### Example 8

The method according to example 7, wherein the third time window ends at a predetermined event or at an end time of the operation.

### Example 9

The method according to example 1, further comprising concealing identifying details of the subject or of the user within the audio segment and the at least one data segment.

### Example 10

The method according to example 1, wherein the at least one data stream comprises data related to actions performed by the user

### Example 11

The method according to example 10, wherein the actions performed by the user comprise user interface activation actions.

### Example 12

The method according to example 1, wherein the at least one data stream comprises data related to actions taken by the system.

### Example 13

The method according to example 12, wherein the actions are taken by the system in response to user actions.

### Example 14

The method according to example 13, wherein the at least one data stream comprises data related to measurements taken by the system.

### Example 15

The method according to example 1, wherein the system is an electrophysiological (EP) mapping and ablation system.

### Example 16

The method according to example 1, wherein the at least one data stream comprises at least one type of data selected from the group consisting of: raw Electrocardiogram (ECG) digital sample recordings collected from ECG electrodes; ECG processed data; Intra-cardiac catheter navigation recordings; Intra-cardiac catheter shaft visualization; catheter localization and visualization algorithmic computation results; localization based on electromagnetic signals; localization based on currents; therapeutic radio frequency ablation or pulse field ablation applications including time duration or repetition information recordings; recording of the measurement of force applied; user action log, including recording of actions performed by the user with a user interface module; keyboard, mouse, or touch screen actions; and ultrasound frames or anatomical markers.

### Example 17

The method according to example 1, wherein the system is an electrophysiological (EP) mapping and ablation system.

### Example 18

The method according to example 1, wherein the operation is a phacoemulsification operation.

### Example 19

A computerized apparatus having a processor coupled with a memory unit, the processor being adapted to perform the steps of: obtaining an audio stream comprising speech by a user of a medical system during an operation involving the medical system, wherein the operation is performed upon a subject; obtaining at least one data stream from equipment associated with the medical system; detecting within the audio stream an indicator to a problem, the indicator occurring at least during a first time window; selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and outputting the audio segment and the at least one data segment.

### Example 20

A computer program product comprising a non-transitory computer readable medium retaining program instructions, which instructions when read by a processor, cause the processor to perform: obtaining an audio stream comprising speech by a user of a medical system during an operation involving the medical system, wherein the operation is performed upon a subject; obtaining at least one data stream from equipment associated with the medical system; detecting within the audio stream an indicator to a problem, the indicator occurring at least during a first time window; selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and outputting the audio segment and the at least one data segment.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method comprising:
obtaining during an operation involving a medical system, an audio stream comprising speech by a user using the medical system for performing the operation, wherein the operation is performed upon a subject;
obtaining at least one data stream from equipment associated with the medical system;
detecting within the audio stream an indicator to a problem, the indicator occurring at least during a time window;
selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and
outputting the audio segment and the at least one data segment.

2. The method of Claim 1, wherein the indicator to the problem is detected by recognizing within the audio stream i) at least one word selected from a predetermined list of words, or ii) an emotional state indicative of a problem.

3. The method of Claim 1, wherein the audio segment and the at least one data segment comprise, respectively, audio and data captured during a second time window preceding the time window.

4. The method of Claim 3, wherein the second time window starts at a predetermined event, or at a beginning time of the operation.

5. The method of Claim 1, wherein the audio segment and the at least one data segment comprise, respectively, audio and data captured during a third time window consecutive to the time window, optionally wherein the third time window ends at a predetermined event or at an end time of the operation.

6. The method of Claim 1, further comprising concealing identifying details of the subject or of the user within the audio segment and the at least one data segment.

7. The method of Claim 1, wherein the at least one data stream comprises data related to actions performed by the user, optionally wherein the actions performed by the user comprise user interface activation actions.

8. The method of Claim 1, wherein the at least one data stream comprises data related to actions taken by the system, optionally wherein the actions are taken by the system in response to user actions, further optionally wherein the at least one data stream comprises data related to measurements taken by the system.

9. The method of Claim 1, wherein the system is an electrophysiological (EP) mapping and ablation system.

10. The method of Claim 1, wherein the at least one data stream comprises at least one type of data selected from the group consisting of: raw Electrocardiogram (ECG) digital sample recordings collected from ECG electrodes; ECG processed data; Intra-cardiac catheter navigation recordings; Intra-cardiac catheter shaft visualization; catheter localization and visualization algorithmic computation results; localization based on electromagnetic signals; localization based on currents; therapeutic radio frequency ablation or pulse field ablation applications including time duration or repetition information recordings; recording of the measurement of force applied; user action log, including recording of actions performed by the user with a user interface module; keyboard, mouse, or touch screen actions; and ultrasound frames or anatomical markers.

11. The method of Claim 1, wherein the operation is a phacoemulsification operation.

12. A computerized apparatus having a processor coupled with a memory unit, the processor being adapted to perform the steps of:
obtaining an audio stream comprising speech by a user of a medical system during an operation involving the medical system, wherein the operation is performed upon a subject;
obtaining at least one data stream from equipment associated with the medical system;
detecting within the audio stream an indicator to a problem, the indicator occurring at least during a first time window;
selecting an audio segment within the audio stream and at least one data segment in each data stream of the at least one data stream the at least one data segment corresponding in time to the audio segment, the audio segment and the at least one data segment comprising audio and data captured, respectively, during at least the time window; and
outputting the audio segment and the at least one data segment.

13. A computerized apparatus having a processor coupled with a memory unit, the processor being adapted to perform the method of any of claims 1 to 11.

14. A computer program product comprising a non-transitory computer readable medium retaining program instructions, which instructions when read by a processor, cause the processor to perform:
obtaining an audio stream comprising speech by a user of a medical system during an operation involving the medical system, wherein the operation is performed upon a subject;
obtaining at least one data stream from equipment associated with the medical system;
detecting within the audio stream an indicator to a problem, the indicator occurring at least during a first time window;
selecting an audio segment within the audio stream and at least one data segment in each data stream of the at least one data stream, the at least one data segment corresponding in time to the audio segment, the audio segment and the at least one data segment comprising audio and data captured, respectively, during at least the time window; and
outputting the audio segment and the at least one data segment.

15. A computer program product comprising a non-transitory computer readable medium retaining program instructions, which instructions when read by a processor, cause the processor to perform the method of any of claims 1 to 11.
